# EUROPEAN PATENT APPLICATION

(11) **EP 4 663 635 A1**
(43) Date of publication of application: **17.12.2025**
(21) Application number: 24752890.4
(22) Date of filing: 07.02.2024
(51) Int. Cl.: C07D 413/14, C07D 498/14, A61K 31/42, A61P 35/00

(54) **CRYSTALLINE FORM OF SULFONAMIDE DERIVATIVE AND PREPARATION METHOD THEREFOR**

(30) Priority: 10.02.2023 CN 202310107569
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: HU, Xiaosi, Shanghai 200245 (CN); YOU, Lingfeng, Shanghai 200245 (CN); FENG, Jun, Shanghai 200245 (CN); HE, Feng, Shanghai 200245 (CN); YANG, Junran, Lianyungang, Jiangsu 222047 (CN); DU, Zhenxing, Lianyungang, Jiangsu 222047 (CN)
(74) Representative: dompatent
(86) International application number: PCT/CN2024/076561
(87) International publication number: WO 2024/165035

(57) **Abstract**

The present disclosure relates to a crystalline form of a sulfonamide derivative and a preparation method therefor. Specifically, the present disclosure provides a new crystalline form of a compound as represented by formula (1) and a preparation method therefor.

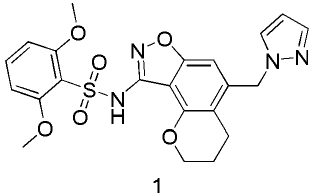

## Description

The present application claims priority to Chinese Patent Application No. 2023101075697 filed on Feb. 10, 2023, which is incorporated herein by reference in its entirety.

### TECHNICAL FIELD

The present disclosure relates to the field of pharmaceutical technology, and in particular, to a crystal form of a sulfonamide derivative and a method for preparing same.

### BACKGROUND

Lysine acetyltransferases (KATs) are a class of enzymes that catalyze the transfer of acetyl group from acetyl-CoA to the ε-amino group of lysine in protein substrates. Lysine acetylation affects protein function and thus plays an important regulatory role in chromosome structures, gene transcription regulation, DNA-binding capacity, enzyme activity and stability, protein interactions, and intracellular localization. KATs are divided into several subfamilies, the biggest of which is MYST (MOZ, YBF2/SAS3, SAS2, TIP60), including KAT5 (TIP60), KAT6A (MOZ; MYST3), KAT6B (MORF; MYST4), KAT7 (HBO; MYST2), and KAT8 (MOF; MYST1). KAT6A/B, as a major member of the MYST family, plays a crucial role in development, stem cell maintenance in the hematopoietic and immune systems, tumor development and progression, and drug resistance.

Patent applications that disclose KAT6 inhibitors include WO2016198507A1, WO2019243491A1, WO2019043139A1, WO2019108824A1, WO2020216701A1, WO2020002587A1, WO2020254946A1, WO2020254989A1, etc.

PCT/CN2022/111395 provides a KAT6 inhibitor, named N-(5-((1H-pyrazol-1-yl)methyl)-3,4-dihydro-2*H*-benzopyran[8,7-*d*]isoxazol-9-yl)-2,6-dimethoxybenzenesulfonamide, with the structure of formula 1, 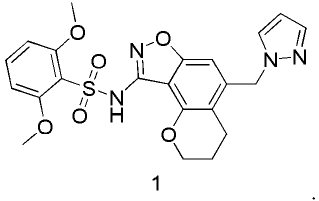 A crystal form used as an active pharmaceutical ingredient often influences the chemical stability of the drug. Variations in crystallization conditions or storage conditions may cause changes in the crystal form structure of a compound and sometimes the generation of other crystal forms. Generally, amorphous drug products have no regular crystal form structure and often have other defects, such as poor product stability, fine particles, difficult filtration, proneness to agglomeration, and poor flowability. The polymorphs of a drug impose different requirements on product storage, production, and scaling up. Therefore, it is necessary to extensively study the crystal forms of the aforementioned compound and to improve the properties of the aforementioned compound.

### SUMMARY

The present disclosure provides a new crystal form of the compound of formula 1, which has good stability and better clinical prospects.

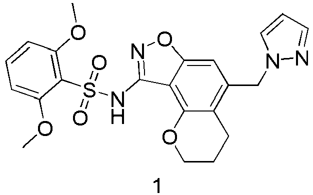

The present disclosure provides a crystal form A of the compound of formula 1 comprising an X-ray powder diffraction pattern represented by diffraction angles 2*θ* having characteristic peaks at 11.288, 16.624, 18.251, 19.639, 22.547, and 26.085.

In some embodiments, the crystal form A of the compound of formula 1 comprises an X-ray powder diffraction pattern represented by diffraction angles 2*θ* having characteristic peaks at 11.288, 16.624, 18.251, 19.639, 21.140, 22.547, 23.439, 26.085, 26.497, and 27.050.

In some embodiments, the crystal form A of the compound of formula 1 comprises an X-ray powder diffraction pattern represented by diffraction angles 2*θ* having characteristic peaks at 11.288, 16.624, 17.312, 18.251, 19.639, 20.086, 21.140, 22.547, 23.439, 24.234, 26.085, 26.497, and 27.050.

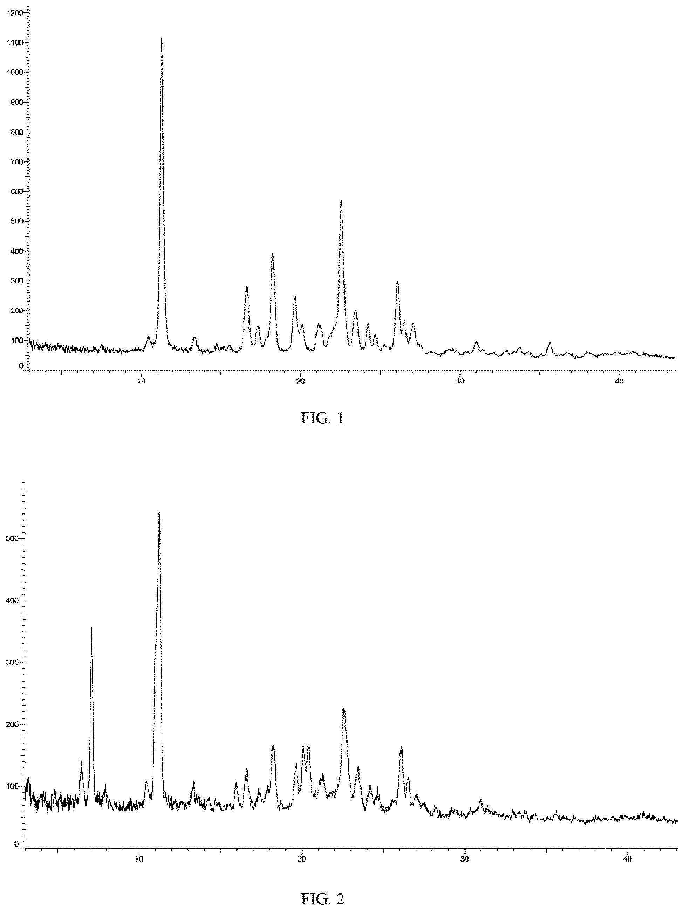

In some embodiments, the crystal form A of the compound of formula 1 comprises an X-ray powder diffraction pattern represented by diffraction angles 2*θ* angles as shown in FIG. 1.

The present disclosure further provides a method for preparing the crystal form A of the compound of formula 1, wherein the method is selected from the group consisting of:
method I, comprising: adding the compound of formula 1 to a solvent (I), and triturating for crystallization, wherein the solvent (I) is one or more solvents selected from the group consisting of an alcohol solvent, a ketone solvent, an ester solvent, an ether solvent, a hydrocarbon solvent, a nitrile solvent, water, and dimethyl sulfoxide; the alcohol solvent is selected from the group consisting of methanol, ethanol, isopropanol, n-propanol, benzyl alcohol, 1,2-propanediol, and isopentanol; the ketone solvent is selected from the group consisting of acetone and methyl isobutyl ketone; the ester solvent is selected from the group consisting of ethyl acetate and isopropyl acetate; the ether solvent is selected from the group consisting of methyl tert-butyl ether, propylene glycol methyl ether, isopropyl ether, and tetrahydrofuran; the hydrocarbon solvent is selected from the group consisting of nitromethane, n-heptane, cyclohexane, toluene, and p-xylene; the nitrile solvent is selected from the group consisting of acetonitrile;
method II, comprising: dissolving the compound of formula 1 in a solvent (II) by heating, and stirring at room temperature or at a reduced temperature for crystallization, wherein the solvent (II) is one or more solvents selected from the group consisting of acetonitrile, nitromethane, benzyl alcohol, N,N-dimethylacetamide, dimethyl sulfoxide, N-methylpyrrolidone, acetonitrile/methanol, and 1,2-dichloroethane; and
method III, comprising: dissolving the compound of formula 1 in a solvent (III), adding a solvent (IV), and stirring for crystallization, wherein the solvent (III) is selected from the group consisting of dimethyl sulfoxide and dichloromethane; the solvent (IV) is one or more solvents selected from the group consisting of water, methanol, ethanol, isopropanol, isopropyl ether, methyl tert-butyl ether, acetone, methyl isobutyl ketone, acetonitrile, ethyl acetate, and toluene.

The present disclosure further provides a crystal form B of the compound of formula 1 comprising an X-ray powder diffraction pattern represented by diffraction angles 2*θ* having characteristic peaks at 7.100, 11.249, 18.241, 22.580, and 26.107.

In some embodiments, the crystal form B of the compound of formula 1 comprises an X-ray powder diffraction pattern represented by diffraction angles 2*θ* having characteristic peaks at 6.454, 7.100, 11.249, 18.241, 19.637, 20.092, 20.407, 22.580, 26.107, and 26.550.

In some embodiments, the crystal form B of the compound of formula 1 comprises an X-ray powder diffraction pattern represented by diffraction angles 2*θ* having characteristic peaks at 6.454, 7.100, 10.448, 11.249, 16.595, 18.241, 19.637, 20.092, 20.407, 22.580, 23.470, 26.107, and 26.550.

In some embodiments, the crystal form B of the compound of formula 1 comprises an X-ray powder diffraction pattern represented by diffraction angles 2*θ* angles as shown in FIG. 2.

The present disclosure further provides a method for preparing the crystal form B, comprising:
dissolving the compound of formula 1 in a solvent (V), adding a solvent (VI), and stirring for crystallization, wherein the solvent (V) is selected from the group consisting of dimethyl sulfoxide; the solvent (VI) is selected from the group consisting of methyl tert-butyl ether.

The present disclosure further provides a crystal form C of the compound of formula 1 comprising an X-ray powder diffraction pattern represented by diffraction angles 2*θ* having characteristic peaks at 6.484, 7.056, 7.907, 11.060, 14.223, and 20.342.

In some embodiments, the crystal form C of the compound of formula 1 comprises an X-ray powder diffraction pattern represented by diffraction angles 2*θ* having characteristic peaks at 6.484, 7.056, 7.907, 11.060, 14.223, 15.973, 20.342, 21.460, 23.090, and 26.398.

In some embodiments, the crystal form C of the compound of formula 1 comprises an X-ray powder diffraction pattern represented by diffraction angles 2*θ* having characteristic peaks at 6.484, 7.056, 7.907, 11.060, 12.702, 14.223, 15.973, 17.526, 20.342, 21.460, 23.090, 26.398, and 27.500.

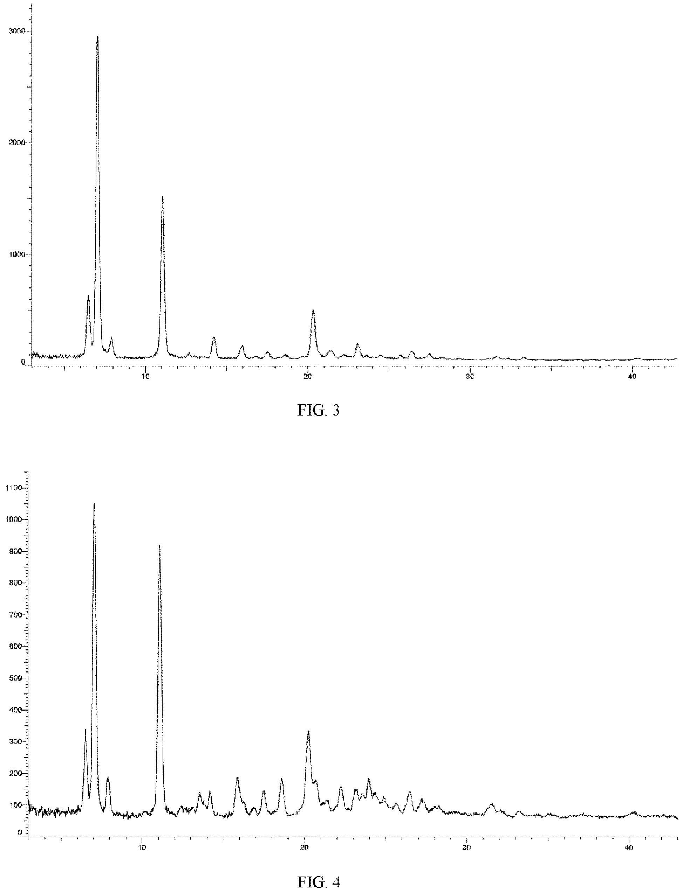

In some embodiments, the crystal form C of the compound of formula 1 comprises an X-ray powder diffraction pattern represented by diffraction angles 2*θ* angles as shown in FIG. 3.

The present disclosure further provides a method for preparing the crystal form C, comprising:
method I, comprising: dissolving the compound of formula 1 in a solvent (VII), and adding a solvent (VIII) for antisolvent crystallization, wherein the solvent (VII) is selected from the group consisting of dichloromethane and tetrahydrofuran, and the solvent (VIII) is one or more solvents selected from the group consisting of tetrahydrofuran and n-hexane; and
method II, comprising: dissolving the compound of formula 1 in a solvent (IX), and stirring for crystallization, wherein the solvent (IX) is selected from the group consisting of trichloromethane.

The present disclosure further provides a crystal form D of the compound of formula 1 comprising an X-ray powder diffraction pattern represented by diffraction angles 2*θ* having characteristic peaks at 6.504, 7.051, 7.896, 11.086, 15.876, and 20.238.

In some embodiments, the crystal form D of the compound of formula 1 comprises an X-ray powder diffraction pattern represented by diffraction angles 2*θ* having characteristic peaks at 6.504, 7.051, 7.896, 11.086, 13.560, 14.194, 15.876, 17.504, 18.599, 20.238, 22.244, and 23.963.

In some embodiments, the crystal form D of the compound of formula 1 comprises an X-ray powder diffraction pattern represented by diffraction angles 2*θ* having characteristic peaks at 6.504, 7.051, 7.896, 11.086, 13.560, 14.194, 15.876, 17.504, 18.599, 20.238, 20.633, 22.244, 23.963, and 26.487.

In some embodiments, the crystal form D of the compound of formula 1 comprises an X-ray powder diffraction pattern represented by diffraction angles 2*θ* angles as shown in FIG. 4.

The present disclosure further provides a method for preparing the crystal form D, comprising:
dissolving the compound of formula 1 in a solvent (X), and stirring for crystallization, wherein the solvent (X) is selected from the group consisting of N,N-dimethylformamide.

The present disclosure further provides a crystal form E of the compound of formula 1 comprising an X-ray powder diffraction pattern represented by diffraction angles 2*θ* having characteristic peaks at 6.490, 7.069, 10.968, 14.194, 16.199, and 20.486.

In some embodiments, the crystal form E of the compound of formula 1 comprises an X-ray powder diffraction pattern represented by diffraction angles 2*θ* having characteristic peaks at 6.490, 7.069, 8.034, 10.968, 14.194, 16.199, 18.656, 20.486, 21.539, and 22.949.

In some embodiments, the crystal form E of the compound of formula 1 comprises an X-ray powder diffraction pattern represented by diffraction angles 2*θ* having characteristic peaks at 6.490, 7.069, 8.034, 10.968, 14.194, 16.199, 18.656, 20.486, 21.539, 22.360, 22.949, 23.979, 24.676, and 26.411.

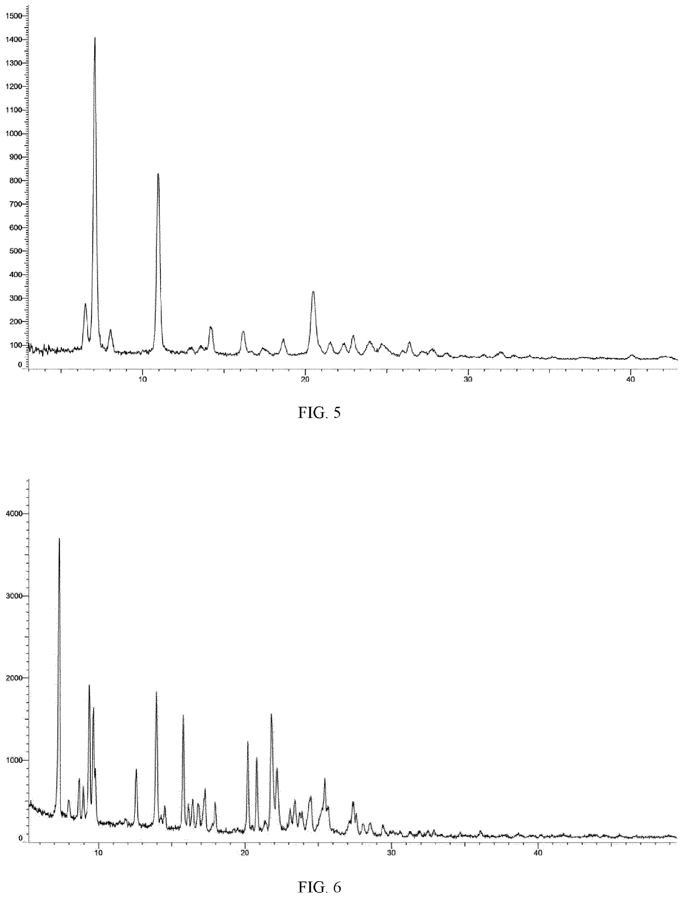

In some embodiments, the crystal form E of the compound of formula 1 comprises an X-ray powder diffraction pattern represented by diffraction angles 2*θ* angles as shown in FIG. 5.

The present disclosure further provides a method for preparing the crystal form E, comprising:
dissolving the compound of formula 1 in a solvent (XI), and stirring for crystallization, wherein the solvent (XI) is selected from the group consisting of 1,2-dichloroethane.

The present disclosure further provides a crystal form F of the compound of formula 1 comprising an X-ray powder diffraction pattern represented by diffraction angles 2*θ* having characteristic peaks at 7.327, 9.387, 13.968, 15.793, 20.198, and 21.813.

In some embodiments, the crystal form F of the compound of formula 1 comprises an X-ray powder diffraction pattern represented by diffraction angles 2*θ* having characteristic peaks at 7.327, 8.690, 9.387, 9.653, 12.583, 13.968, 15.793, 17.270, 20.198, 20.810, 21.813, 22.186, and 25.444.

In some embodiments, the crystal form F of the compound of formula 1 comprises an X-ray powder diffraction pattern represented by diffraction angles 2*θ* having characteristic peaks at 7.327, 8.690, 8.970, 9.387, 9.653, 12.583, 13.968, 15.793, 16.429, 17.270, 17.975, 20.198, 20.810, 21.813, 22.186, 23.410, 24.438, 25.444, and 27.356.

In some embodiments, the crystal form F of the compound of formula 1 comprises an X-ray powder diffraction pattern represented by diffraction angles 2*θ* angles as shown in FIG. 6.

The present disclosure further provides a method for preparing the crystal form F, comprising: dissolving the compound of formula 1 in a solvent (XII), and stirring for crystallization, wherein the solvent (XII) is selected from the group consisting of ethyl acetate.

In some embodiments, the crystal form G of the compound of formula 1 comprises an X-ray powder diffraction pattern represented by diffraction angles 2*θ* having characteristic peaks at 6.614, 7.192, 8.164, 11.113, 16.302, and 20.560.

In some embodiments, the crystal form G of the compound of formula 1 comprises an X-ray powder diffraction pattern represented by diffraction angles 2*θ* having characteristic peaks at 6.614, 7.192, 8.164, 11.113, 11.377, 12.978, 14.360, 16.302, 20.560, 21.639, 22.588, 23.042, 24.186, and 26.521.

In some embodiments, the crystal form G of the compound of formula 1 comprises an X-ray powder diffraction pattern represented by diffraction angles 2*θ* having characteristic peaks at 6.614, 7.192, 8.164, 11.113, 11.377, 12.266, 12.978, 13.738, 14.360, 16.302, 18.807, 20.560, 21.639, 22.588, 23.042, 24.186, 26.080, and 26.521.

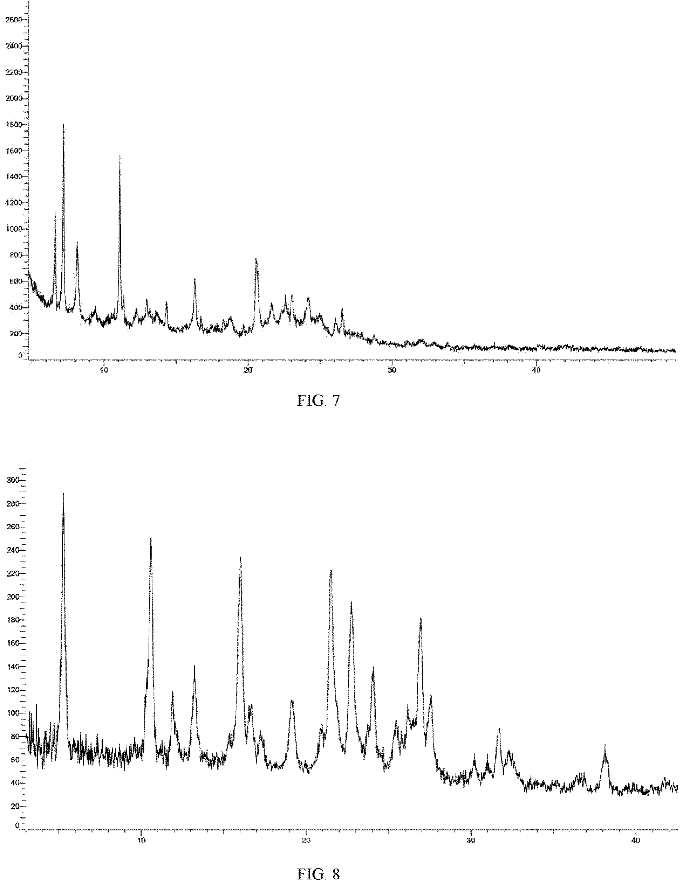

In some embodiments, the crystal form G of the compound of formula 1 comprises an X-ray powder diffraction pattern represented by diffraction angles 2*θ* angles as shown in FIG. 7.

The present disclosure further provides a method for preparing the crystal form G, comprising: dissolving the compound of formula 1 in a solvent (XIII), and volatilizing the solvent for crystallization, wherein the solvent (XIII) is selected from the group consisting of dichloromethane.

The present disclosure further provides a crystal form H of the compound of formula 1 comprising an X-ray powder diffraction pattern represented by diffraction angles 2*θ* having characteristic peaks at 5.271, 10.587, 13.230, 16.017, 21.498, 22.753, and 26.938.

In some embodiments, the crystal form H of the compound of formula 1 comprises an X-ray powder diffraction pattern represented by diffraction angles 2*θ* having characteristic peaks at 5.271, 10.587, 11.892, 13.230, 16.017, 16.625, 19.110, 21.498, 22.753, 24.080, 26.938, and 27.536.

In some embodiments, the crystal form H of the compound of formula 1 comprises an X-ray powder diffraction pattern represented by diffraction angles 2*θ* having characteristic peaks at 5.271, 10.587, 11.892, 13.230, 16.017, 16.625, 19.110, 21.498, 22.753, 24.080, 25.459, 26.175, 26.938, 27.536, 31.694, and 38.116.

In some embodiments, the crystal form H of the compound of formula 1 comprises an X-ray powder diffraction pattern represented by diffraction angles 2*θ* angles as shown in FIG. 8.

The present disclosure further provides a method for preparing the crystal form H, comprising:
a) mixing the compound of formula 1 with an aqueous sodium hydroxide solution;
b) stirring at 37 °C to precipitate a solid; and
c) discarding the supernatant, adding an aqueous hydrochloric acid solution for resuspension and trituration at 37 °C, centrifuging, and drying in vacuum to give a solid.

In some embodiments, the crystal form I of the compound of formula 1 comprises an X-ray powder diffraction pattern represented by diffraction angles 2*θ* having characteristic peaks at 5.963, 11.883, 13.953, 19.518, 20.305, and 22.086.

In some embodiments, the crystal form I of the compound of formula 1 comprises an X-ray powder diffraction pattern represented by diffraction angles 2*θ* having characteristic peaks at 5.963, 9.438, 10.303, 11.883, 13.953, 17.945, 18.356, 19.518, 19.807, 20.305, 22.086, 25.009, and 29.576.

In some embodiments, the crystal form I of the compound of formula 1 comprises an X-ray powder diffraction pattern represented by diffraction angles 2*θ* having characteristic peaks at 5.963, 9.438, 10.303, 11.156, 11.883, 13.446, 13.953, 17.945, 18.356, 19.518, 19.807, 20.305, 21.559, 22.086, 25.009, 29.576, and 30.192.

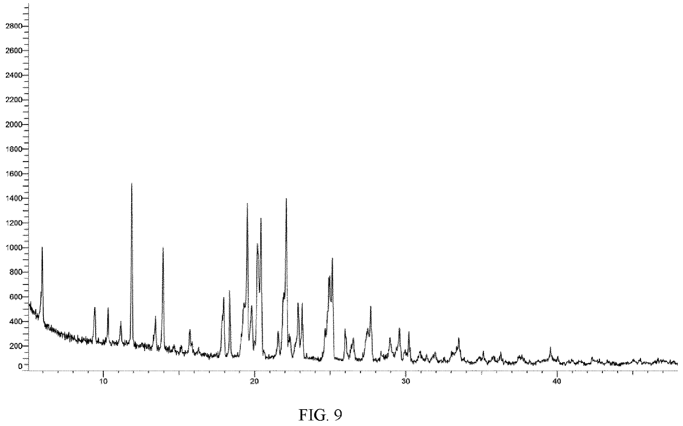

In some embodiments, the crystal form I of the compound of formula 1 comprises an X-ray powder diffraction pattern represented by diffraction angles 2*θ* angles as shown in FIG. 9.

The present disclosure further provides a method for preparing the crystal form I, comprising: dissolving the compound of formula 1 in a solvent (XIV), and volatilizing the solvent for crystallization, wherein the solvent (XIV) is selected from the group consisting of dichloromethane.

The present disclosure further provides a pharmaceutical composition comprising the aforementioned crystal form A, crystal form B, crystal form C, crystal form D, crystal form E, crystal form F, crystal form G, crystal form H, or crystal form I, and optionally a pharmaceutically acceptable auxiliary material selected from the group consisting of a pharmaceutically acceptable excipient.

The present disclosure further provides a pharmaceutical composition prepared from the aforementioned crystal form A, crystal form B, crystal form C, crystal form D, crystal form E, crystal form F, crystal form G, crystal form H, or crystal form I, and optionally a pharmaceutically acceptable excipient.

The present disclosure further provides a method for preparing a pharmaceutical composition, comprising: mixing the aforementioned crystal form A, crystal form B, crystal form C, crystal form D, crystal form E, crystal form F, crystal form G, crystal form H, or crystal form I with a pharmaceutically acceptable excipient.

The present disclosure further provides use of the aforementioned crystal form A, crystal form B, crystal form C, crystal form D, crystal form E, crystal form F, crystal form G, crystal form H or crystal form I, or the aforementioned composition in the manufacture of a medicament for preventing and/or treating cancer.

According to the use of the present disclosure, the cancer is selected from the group consisting of breast cancer, ovarian cancer, pancreatic cancer, prostate cancer, gastric cancer, colorectal cancer, lung cancer, renal cancer, hepatic cancer, cervical cancer, endometrial cancer, myeloma, leukemia, lymphoma, acoustic neuroma, basal cell carcinoma, cholangiocarcinoma, bladder cancer, brain cancer, bronchogenic carcinoma, sarcoma, chordoma, choriocarcinoma, craniopharyngioma, cystadenocarcinoma, embryonal carcinoma, hemangioendothelioma, ependymoma, epithelial cancer, esophageal cancer, essential thrombocytosis, Ewing sarcoma, testicular cancer, glioma, heavy chain disease, hemangioblastoma, medullary carcinoma, medulloblastoma, melanoma, meningioma, mesothelioma, neuroblastoma, NUT midline carcinoma, neuroglioma, bone cancer, nasopharyngeal carcinoma, oral cancer, thyroid cancer, pinealoma, polycythemia vera, retinoblastoma, sebaceous carcinoma, seminoma, skin cancer, squamous cell carcinoma, synovioma, sweat gland carcinoma, Waldenström macroglobulinemia, and Wilms tumor; preferably, the cancer is selected from the group consisting of breast cancer, ovarian cancer, pancreatic cancer, prostate cancer, gastric cancer, colorectal cancer, and lung cancer.

As used herein, the "2*θ* or 2*θ* angle" refers to a diffraction angle; θ refers to the Bragg angle in ° or degrees; the 2*θ* of each characteristic peak has a margin of error of ±0.20 (including the case that a number having more than 1 decimal place is rounded), specifically -0.20, -0.19, - 0.18, -0.17, -0.16, -0.15, -0.14, -0.13, -0.12, -0.11, -0.10, -0.09, -0.08, -0.07, -0.06, -0.05, - 0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, numerical values such as the content of related substances are data obtained by measurement and calculation, and there is inevitably a certain degree of error. In general, ±10% is a reasonable margin of error. There is a certain degree of error change depending on the context where it is used, and the error change is no more than ±10% and may be ±9%, ±8%, ±7%, ±6%, ±5%, ±4%, ±3%, ±2%, or ±1%, preferably ±5%.

The starting material used in the methods for preparing the crystal forms of the present disclosure may be any form of the compound, and the specific forms include, but are not limited to: an amorphous form, any crystal form, a hydrate, a solvate, and the like.

In the present disclosure, the drying temperature is generally 25 °C-100 °C, preferably 40 °C-70 °C, and the drying may be performed at an atmospheric pressure or reduced pressure.

Methods for the crystallization described in the present disclosure include room-temperature crystallization, cooling crystallization, crystallization by volatilizing the solvent, crystallization induced by adding a seed crystal, and the like, wherein the cooling temperature is selected from the group consisting of 65 °C or lower, preferably from the group consisting of -10 °C to 60 °C, and the crystallization process may involve stirring.

As used herein, the "differential scanning calorimetry" or "DSC" refers to the measurement of the temperature difference and heat flow difference between a sample and a reference substance during a ramping or thermostatic process to characterize all the physical changes and chemical changes related to the thermal effect, and to acquire phase change information about the sample.

According to the description of hygroscopicity characteristics and the definitions of hygroscopic weight gains in "General Rule 9103, Guidelines for Hygroscopicity" in the Chinese Pharmacopoeia, Volume IV, 2015 Edition,
deliquescent: sufficient water is absorbed to form a liquid;
extremely hygroscopic: the hygroscopic weight gain is not less than 15%;
hygroscopic: the hygroscopic weight gain is less than 15% but not less than 2%;
slightly hygroscopic: the hygroscopic weight gain is less than 2% but not less than 0.2%;
nonhygroscopic or practically nonhygroscopic: the hygroscopic weight gain is less than 0.2%.

As used herein, the "excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows an XRPD pattern of the crystal form A of compound 1.
FIG. 2 shows an XRPD pattern of the crystal form B of compound 1.
FIG. 3 shows an XRPD pattern of the crystal form C of compound 1.
FIG. 4 shows an XRPD pattern of the crystal form D of compound 1.
FIG. 5 shows an XRPD pattern of the crystal form E of compound 1.
FIG. 6 shows an XRPD pattern of the crystal form F of compound 1.
FIG. 7 shows an XRPD pattern of the crystal form G of compound 1.
FIG. 8 shows an XRPD pattern of the crystal form H of compound 1.
FIG. 9 shows an XRPD pattern of the crystal form I of compound 1.

### DETAILED DESCRIPTION

The present disclosure is explained below in greater detail with reference to examples or experimental examples. The examples or experimental examples in the present disclosure are only illustrative of the technical solutions in the present disclosure and do not limit the spirit and scope of the present disclosure.

Test conditions for the instruments used in the experiments:
The structures of the compounds were determined by nuclear magnetic resonance (NMR) spectroscopy or/and mass spectrometry (MS). The NMR shifts (δ) are given in 10⁻⁶ (ppm). The NMR analyses were performed using a Bruker AVANCE NEO 500M nuclear magnetic resonance system, with dimethyl sulfoxide-D6 (DMSO-*d*₆), chloroform-D (CDCl₃), and methanol-D4 (CD₃OD) as solvents and tetramethylsilane (TMS) as an internal standard.

The MS analyses were performed using an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector) and THERMO Ultimate 3000-Q Exactive (manufacturer: THERMO; MS model: THERMO Q Exactive).

The high performance liquid chromatography (HPLC) analyses were performed using Agilent HPLC 1200DAD, Agilent HPLC 1200VWD, and a Waters HPLC e2695-2489 high performance liquid chromatograph.

The preparative high performance liquid chromatography steps were performed using Waters 2767, Waters 2767-SQ Detecor2, Shimadzu LC-20AP, and a Gilson-281 preparative chromatograph.

The silica gel column chromatography generally used 200-300 mesh Yantai Huanghai silica gel as the support.

XRPD refers to X-ray powder diffraction: The analysis was performed using a BRUKER D8 X-ray diffractometer; specific acquisition information: a Cu anode (40 kV, 40 mA), Cu-Kα1 ray (λ = 1.54060 Å), Kα2 ray (λ = 1.54439 Å), and Kβ ray (λ = 1.39222 Å). Scan mode: *θ*/2*θ*, scan range (2*θ* range): 5° to 45°.

DSC refers to differential scanning calorimetry: The analysis was performed using a METTLER TOLEDO DSC 3+ differential scanning calorimeter, with a temperature ramp rate of 10 °C/min, specific temperature ranges shown in corresponding patterns (mostly 25-350 °C), and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding patterns (mostly 25-350 °C), and a nitrogen purge speed of 50 mL/min.

DVS refers to dynamic vapor sorption: The analysis was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criterion was dm/dt being not greater than 0.002%.

The known starting materials in the present disclosure may be synthesized by using or according to methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed in an argon atmosphere or a nitrogen atmosphere unless otherwise specified.

The argon atmosphere or nitrogen atmosphere means that the reaction flask was connected to a balloon containing about 1 L of argon or nitrogen gas.

The monitoring of the reaction progress in the examples was performed by thin-layer chromatography (TLC). The developing solvents for the reactions, the eluent systems for the column chromatography purification, and the developing solvent systems for the thin-layer chromatography include: A: a n-hexane/ethyl acetate system, and B: a dichloromethane/methanol system. The volume ratio of the solvents was adjusted depending on the polarity of the compound, or small amounts of basic or acidic reagents such as triethylamine and acetic acid may also be added for adjustments.

### Example 1: Preparation of compound of formula 1

### N-(5-((1H-pyrazol-1-yl)methyl)-3,4-dihydro-2H-chromeno[8,7-d]isoxazol-9-yl)-2,6-dimethoxybenzenesulfonamide 1

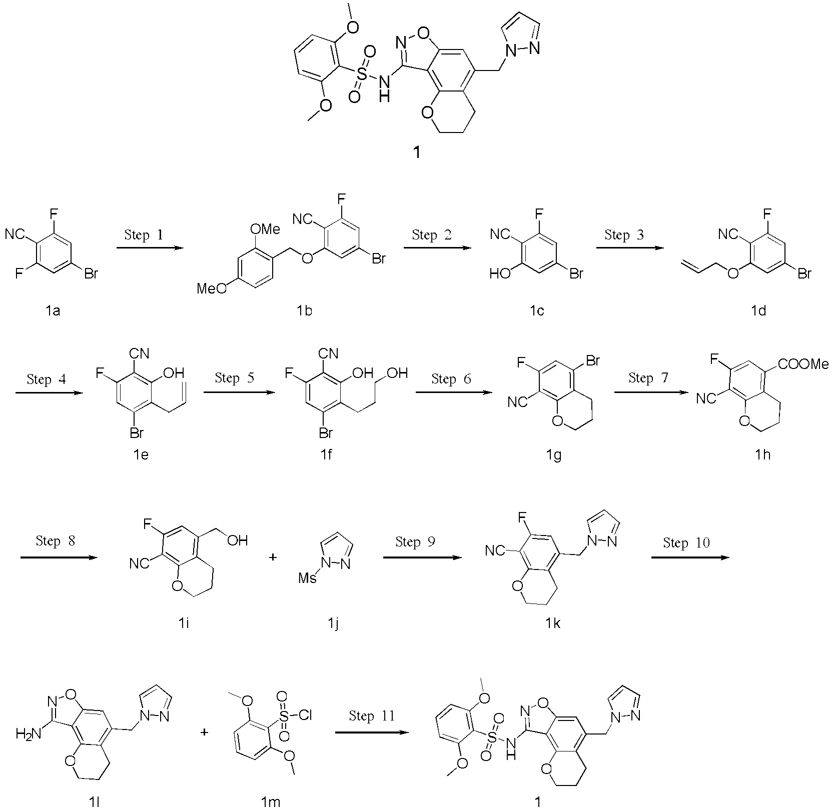

### Step 1

### 4-Bromo-2-((2,4-dimethoxybenzyl)oxy)-6-fluorobenzonitrile 1b

4-Bromo-2,6-difluorobenzonitrile **1a** (22 g, 101 mmol, Accela) and 2,4-dimethoxybenzyl alcohol (18.5 g, 110 mmol, Bidepharm) were dissolved in *N,N*-dimethylformamide (200 mL), and cesium carbonate (49 g, 150 mmol, Accela) was added. The reaction mixture was stirred at 60 °C for 16 h. The reaction mixture was cooled to room temperature and filtered at reduced pressure. The filtrate was diluted with ethyl acetate (500 mL) and washed with a saturated sodium chloride solution (30 mL × 5). The resulting organic phase was dried over anhydrous sodium sulfate and filtered, and the filtrate was concentrated at reduced pressure to give the title product **1b** (36.9 g, yield: 100%). The product was directly used in the next step without purification.

### Step 2

### 4-Bromo-2-fluoro-6-hydroxybenzonitrile 1c

Compound **1b** (36.9 g, 100.7 mmol) was dissolved in dichloromethane (250 mL), and the solution was cooled to 0 °C. Trifluoroacetic acid (39 g, 342 mmol, Adamas) was added dropwise, and the reaction mixture was warmed to room temperature and stirred for 1 h. The reaction mixture was concentrated at reduced pressure, and the resulting residue was purified by silica gel column chromatography with eluent system A to give the title product **1c** (9.7 g, yield: 44.5%).

### Step 3

### 2-(Allyloxy)-4-bromo-6-fluorobenzonitrile 1d

Compound **1c** (10.7 g, 49.5 mmol) was dissolved in *N,N-d*imethylformamide (120 mL), and the reaction mixture was cooled to 0 °C. Cesium carbonate (24 g, 73.7 mmol, Bidepharm) and allyl bromide (11.2 g, 92.6 mmol, Adamas) were added, and the reaction mixture was warmed to room temperature and stirred for 4 h. The reaction mixture was filtered at reduced pressure. The filtrate was diluted with ethyl acetate (400 mL) and washed with a saturated sodium chloride solution (30 mL × 3). The resulting organic phase was dried over anhydrous sodium sulfate and filtered, and the filtrate was concentrated at reduced pressure. The resulting residue was purified by silica gel column chromatography with eluent system A to give the title product **1d** (11.7 g, yield: 92%). ¹H NMR (500 MHz, CDCl₃) δ 7.02 (dt, 1H), 6.95 (t, 1H), 6.04 (m, 1H), 5.57-5.47 (m, 1H), 5.41 (dt, 1H), 4.75-4.64 (m, 2H).

### Step 4

### 3-Allyl-4-bromo-6-fluoro-2-hydroxybenzonitrile 1e

Compound **1d** (3.35 g, 13.1 mmol) was dissolved in 1,2-dichlorobenzene (80 mL), and the reaction mixture was purged with nitrogen three times and stirred at 180 °C for 13 h. The reaction mixture was cooled to room temperature, and the resulting residue was purified by silica gel column chromatography (wet loading) with eluent system A to give the title product **1e** (2.77 g, yield: 82.7%). ¹H NMR (500 MHz, CDCl₃) δ 7.07 (dd, 1H), 6.45 (s, 1H), 5.90 (dddd, 1H), 5.24-5.10 (m, 2H), 3.68-3.55 (m, 2H).

### Step 5

### 4-Bromo-6-fluoro-2-hydroxy-3-(3-hydroxypropyl)benzonitrile 1f

Compound **1e** (4.8 g, 18.7 mmol) was dissolved in anhydrous tetrahydrofuran (100 mL), and a solution of borane in tetrahydrofuran (1.0 M, 22 mL, 22 mmol, Adamas) was added dropwise at 0 °C. The reaction mixture was stirred in an ice bath for 2 h. A 3 M aqueous solution of sodium hydroxide (13 mL, 39 mmol) and 30% hydrogen peroxide (3.0 mL) were sequentially added in an ice-bath condition. After the addition, the mixture was stirred for 10 min. The pH of the reaction mixture was adjusted to 2 with 2 M hydrochloric acid, and the reaction mixture was extracted with ethyl acetate (100 mL × 2). The organic phase was collected, dried over anhydrous sodium sulfate, and filtered. The filtrate was concentrated at reduced pressure, and the resulting residue was purified by silica gel column chromatography with eluent system A to give the title product **1f** (3.5 g, yield: 68.1%). MS m/z (ESI): 275.8 [M+1]. ¹H NMR (500 MHz, CDCl₃) δ 7.04 (d, 1H), 3.71 (t, 2H), 3.00-2.98 (m, 2H), 2.01-1.96 (m, 2H).

### Step 6

### 5-Bromo-7-fluorochromane-8-carbonitrile 1g

Compound **1f** (3.8 g, 13.9 mmol) was dissolved in anhydrous tetrahydrofuran (80 mL), and the reaction was cooled to 0 °C. Triphenylphosphine (4.4 g, 16.8 mmol, Sinopharm) and diisopropyl azodicarboxylate (3.4 g, 16.8 mmol, Accela) were added, and the reaction mixture was warmed to room temperature and stirred for 2 h. The reaction mixture was concentrated at reduced pressure, and the resulting residue was purified by silica gel column chromatography with eluent system A to give the title product **1g** (3.0 g, yield: 84.5%). ¹H NMR (500 MHz, CDCl₃) δ 7.03 (d, 1H), 4.33 (t, 2H), 2.77-2.74 (m, 2H), 2.12-2.08 (m, 2H). MS m/z (ESI): 257.8 [M+1].

### Step 7

### Methyl 8-cyano-7-fluorochromane-5-carboxylate 1h

Compound **1g** (2.6 g, 10.2 mmol) was dissolved in 40 mL of a mixed solvent of methanol and *N,N*-dimethylformamide (V:V = 1:3), and [1,1'-bis(diphenylphosphino)ferrocene]palladium dichloride (800 mg, 1.09 mmol, Adamas) and triethylamine (3.0 g, 2.93 mmol, Sinopharm) were sequentially added. The mixture was purged with carbon monoxide 3 times and stirred at 90 °C at 10 bar pressure for 16 h. The reaction mixture was cooled to room temperature, concentrated at reduced pressure, diluted with ethyl acetate (150 mL), and washed with a saturated sodium chloride solution (50 mL × 3). The resulting organic phase was dried over anhydrous sodium sulfate and filtered, and the filtrate was concentrated at reduced pressure. The resulting residue was purified by silica gel column chromatography with eluent system A to give the title product **1h** (2.1 g, yield: 87.9%). ¹H NMR (500 MHz, CDCl₃) δ 7.26 (d, 1H), 4.38-4.36 (m, 2H), 3.93 (s, 3H), 3.10-3.07 (m, 2H), 2.08-2.03 (m, 2H). MS m/z (ESI): 235.9 [M+1].

### Step 8

### 7-Fluoro-5-(hydroxymethyl)chromane-8-carbonitrile 1i

Compound **1h** (2.1 g, 8.93 mmol) was dissolved in dry tetrahydrofuran (40 mL), and the reaction mixture was purged with nitrogen 3 times and cooled to 0 °C. Lithium borohydride (2 M, 18 mmol, 9.0 mL, Adamas) was added. The reaction mixture was heated to 70 °C and stirred for 2 h. The reaction mixture was cooled to room temperature, quenched with water (1 mL), diluted with ethyl acetate (100 mL), and washed with a saturated sodium chloride solution (50 mL × 2). The resulting organic phase was dried over anhydrous sodium sulfate and filtered, and the filtrate was concentrated at reduced pressure. The resulting residue was purified by silica gel column chromatography with eluent system A to give the title product **1i** (1.84 g, yield: 99.5%). MS m/z (ESI): 207.9 [M+1].

### Step 9

### 5-((1H-pyrazol-1-yl)methyl)-7-fluorochromane-8-carbonitrile 1k

Compound **1i** (1.8 g, 8.69 mmol) and 1-(methylsulfonyl)-1*H*-pyrazole **1j** (1.5 g, 10.3 mmol, prepared by the method for "intermediate 13 disclosed in scheme 8 on page 63 of the specification of the patent application WO2020254946A1") were dissolved in acetonitrile (30 mL), and cesium carbonate (4.2 g, 12.9 mmol, Accela) was added. The mixture was left to react at 70 °C for 1 h. The reaction mixture was filtered, and the filtrate was concentrated at reduced pressure. The resulting residue was purified by silica gel column chromatography with eluent system A to give the title product **1k** (1.9 g, yield: 85.0%). MS m/z (ESI): 258.0 [M+1].

### Step 10

### 5-((1H-pyrazol-1-yl)methyl)-3,4-dihydro-2H-chromeno[8,7-d]isoxazol-9-amine 1l

Compound **1k** (1.9 g, 7.39 mmol) and acetohydroxamic acid (1.7 g, 22.2 mmol, Adamas) were dissolved in *N,N-*dimethylformamide (30 mL) and water (4.0 mL), and potassium carbonate (6.2 g, 44.9 mmol, Sinopharm) was added. The reaction mixture was stirred at 70 °C for 24 h. The reaction mixture was cooled to room temperature, and water (100 mL) was added. The mixture was filtered, and the filter cake was collected and dried to give the title product **1l** (1.65 g, yield: 82.7%). ¹H NMR (500 MHz, DMSO-*d*₆) δ 7.78 (d, 1H), 7.52 (d, 1H), 6.34 (s, 1H), 6.32 (t, 1H), 5.85 (s, 2H), 5.39 (s, 2H), 4.25-4.23 (m, 2H), 2.68 (t, 2H), 2.03-1.98 (m, 2H). MS m/z (ESI): 271.0 [M+1].

### Step 11

### N-(5-((1H-pyrazol-1-yl)methyl)-3,4-dihydro-2H-chromeno[8,7-d]isoxazol-9-yl)-2,6-dimethoxybenzenesulfonamide 1

Compound **1l** (200 mg, 0.740 mmol) and compound **1m** (300 mg, 1.27 mmol) were dissolved in pyridine (5.0 mL), and the solution was purged with nitrogen 3 times. The reaction mixture was microwaved at 120 °C for 3 h. The reaction mixture was cooled to room temperature and concentrated at reduced pressure. The resulting residue was purified by high performance liquid chromatography (Xtimate phenyl-hexyl Prep C18 5 µm, 30 × 150 mm; mobile phases: A-aqueous phase (0.1% ammonia water): B-acetonitrile = 5%-45% (20 min), flow rate: 30 mL/min) to give the title product **1** (40 mg, yield: 11.5%). ¹H NMR (500 MHz, DMSO-*d*₆) δ 9.40 (s, 1H), 7.79 (d, 1H), 7.52 (d, 1H), 7.48 (t, 1H), 6.77 (d, 2H), 6.43 (s, 1H), 6.32 (t, 1H), 5.42 (s, 2H), 4.25 (t, 2H), 3.78 (s, 6H), 2.70 (t, 2H), 2.04-1.99 (m, 2H). MS m/z (ESI): 470.8 [M+1].

### Test Example 1. Enzymatic activity assay of compound of formula 1 KAT6 (AlphaScreen Method)

### I. Materials

1. KAT6A (customized by Chempartner)
2. Ovalbumin (Sigma-Aldrich, A5378-5G)
3. 2 M Tris-HCl solution, pH 7.8, sterile (Sangon, B548140-0500)
4. 5 M NaCl solution (Sangon, B548121-0100)
5. EDTA (0.5 M), pH 8.0, RNase-free (Thermofisher, AM9260G)
6. Tween-20 (Sangon, A100777-0500)
7. DTT, 1 M (Invitrogen, P2325)
8. Acetyl coenzyme A (Ac-CoA, CAYMAN, Cat. No. 16160)
9. Recombinant histone H3.1 biotinylated (human) (Active Motif 31696)
10. 384-well plate, light gray (PerkinElmer, Cat. No. 6005350)
11. Anacardic acid (MCE, Cat. No. HY-N2020)
12. AlphaScreen streptavidin donor beads, 5 mg (PerkinElmer, 6760002)
13. AlphaScreen protein A acceptor beads, 5 mg (PerkinElmer, 6760137M)
14. Acetylated-lysine antibody #9441 (CST 9441S)
15. PHERA star microplate reader (BMG labtech)

### II. Methodology

### 1. Reagent preparation

a. 1× assay buffer: 100 mM Tris-HCl, pH 7.8; 15 mM NaCl; 1 mM EDTA; 0.01% tween-20; 1 mM DTT; 0.01% m/v ovalbumin.
b. KAT enzyme solution: 1.25 nM (final concentration), prepared in 1× assay buffer.
c. Mixed substrate of Ac-CoA and H3: a mixed substrate of 1000 nM (final concentration) Ac-CoA and 55 nM (final concentration) H3, prepared in 1× assay buffer.
d. Compounds: initial concentration of 100 µM, 3-fold dilution, 10 serial concentrations. All compound concentrations were diluted 83-fold with 1× assay buffer for later use.
e. Assay reagent: 8 ng/µL (final concentration) AlphaScreen protein A acceptor beads, 8 ng/µL AlphaScreen streptavidin donor beads, 1: 1500 diluted acetylated-lysine antibody, and 100 µM anacardic acid; prepared in 1× assay buffer.

### 2. Procedures

The prepared enzyme solution was added to a 384-well plate at 3 µL/well, and 3 µL of 1× assay buffer was added to each well in columns 23 and 24 (Min). 3 µL of compound solution was added to each well, and 3 µL of buffer was added to each Min well; 3 µL of DMSO solution was added to each well in columns 1 and 2 (Max) as controls. The plate was centrifuged, shaken for 2 min, and incubated at room temperature for 15 min. The mixed substrate of Ac-CoA and H3 was added at 6 µL/well, and the plate was centrifuged, shaken for 2 min, and incubated at room temperature for 20 min. The assay reagent was added at 6 µL/well, and the plate was centrifuged, shaken for 2 min, and incubated in the dark at room temperature for 120 min. Plate reading was performed on the microplate reader, and AlphaScreen counts were recorded. Plotting was performed using the Graphpad software, and the IC₅₀ values of the compounds were calculated. The result was IC ₅₀ = 0.3 nM.

Conclusion: The compound of formula 1 has a good inhibitory effect on KAT6A.

### Test Example 2. Anti-ZR-75-1 proliferation assay of compound of formula 1

### I. Reagents and instruments

1. ZR-75-1 (ATCC CRL1500)
2. 1640 medium (Gibco, 22400-089)
3. 0.25% trypsin-EDTA (1×) (Gibco, 25200-072)
4. Penicillin-streptomycin (Gibco, 15140-122)
5. DPBS (1×) (Gibco, 14190-144)
6. FBS (Gibco, 10091148)
7. Assay plate, 96-well, black with clear bottom (Corning, 3603)
8. 96-well non-treated round-bottom plate (JET BIOFIL, TCP-002-096)
9. CellTiter-Glo buffer (Promega, G756B)
10. CellTiter-Glo substrate (Promega, G755B)
11. Automatic cell counter (Countstar, IC1000)
12. Thermostatic incubator (Thermo, I160)
13. PHERAstar FS (BMG labtech, PHERAstar FS)

### II. Methodology

### 1. Cell plating (day 0)

a. The state of cells was observed under a microscope to make sure that the cell confluence was about 90%.
b. The cell supernatant was discarded. The cells were rinsed with DPBS once, and the DPBS was removed. The cells were digested with a proper amount of trypsin and left to stand at 37 °C for 5 min.
c. The digestion was stopped with an equal volume of 1640 medium containing 10% FBS, and the cell suspension was collected and centrifuged at 300 g for 3 min. The cells were suspended in a proper amount of fresh medium.
d. The resulting cell suspension was collected and cell counting was performed.
e. The cell suspension was diluted to 5 × 10⁴ cells/mL, 50 µL/well with 1640 medium containing 10% FBS. Each well contained 2500 ZR-75-1 cells.
f. The cell culture plates were incubated overnight in an incubator at 37 °C and 5% carbon dioxide.

### 2. Compound addition (day 1)

a. Each compound was serially diluted in DMSO to 9 concentration points (initial concentration of 100 µM, 3-fold dilution; the highest concentration of each compound may be adjusted depending on its IC₅₀). For example, in a 96-well non-treated round-bottom plate, 3 µL of compound was serially diluted in 6 µL of DMSO.
b. Each concentration point of each compound was diluted 500-fold in the corresponding volume of 1640 medium.
c. 50 µL of diluted compound solution was added to the cell supernatant (50 µL/well) in each cell plate.
d. After the addition, the cell plates were incubated in an incubator at 37 °C and 5% carbon dioxide.

### 3. Re-digestion, plating, and compound addition (day 7)

a. Six days after the addition, the compound-containing medium was discarded. Then the cells were rinsed with DPBS (150 µL/well) once, and the DPBS was immediately pipetted off.
b. The cells were digested with 50 µL of trypsin and left to stand at 37 °C for 3 min, and then the digestion was stopped with 1640 medium containing 10% FBS (150 µL/well).
c. The cells were well mixed using a pipette and re-plated at a ratio of 1:8; that is, 25 µL of cell suspension was pipetted into a new 96-well plate (25 µL of 1640 medium containing 10% FBS had been added to the new plate in advance).
d. Compound solution preparation and addition (50 µL/well) were performed according to steps a to c in 2.
e. After the addition, the cell plates were incubated in an incubator at 37 °C and 5% carbon dioxide.

### 4. CTG assay (day 14)

a. CellTiter-Glo buffer and lyophilized CellTiter-Glo substrate were equilibrated to room temperature before use and were well mixed to prepare a 100-mL CellTiter-Glo reagent (or a prepared CellTiter-Glo reagent was removed from a -20 °C freezer and equilibrated to room temperature).
b. The plates to be tested were removed from the incubator and equilibrated to room temperature, and the CellTiter-Glo reagent was added at 50 µL/well.
c. The plates were shaken for 2 min so that the cells were completely lysed.
d. After the plates were left to stand at room temperature for 28 min and signals were stable, the plates were tested on PHERAstar FS.

**Table 1. IC₅₀ and maximum inhibition rate for inhibition of ZR-75-1 proliferation by compound of formula 1**

| Compound | ZR-75-1/IC₅₀ (nM) | Maximum inhibition rate/Imax% |
|---|---|---|
| Compound of formula 1 | 0.4 | 96 |

Conclusion: The compound of formula 1 has a good inhibitory effect on ZR-75-1 proliferation.

### Example 2. Preparation of crystal form A of compound of formula 1

10 mg of the compound of formula 1 was added to 1 mL of the solvent. The mixture was heated or cooled to 60 °C to 5 °C at ±0.75 °C/min, stirred, triturated, and filtered. The filter cake was collected and dried in vacuum to give a solid. The product was identified by X-ray powder diffraction as crystal form A. The solvents are shown in Table 2 below. The XRPD pattern is shown in FIG. 1, with its characteristic peak positions listed in Table 3. The DSC profile shows an endothermic peak at 240.55 °C. The TGA profile of the crystal form suggests substantially no weight loss from 30 °C to 267 °C.

**Table 2. Solvents selected for preparing crystal form A by triturating for crystallization and XRPD results**

| Solvent | Crystal form |
|---|---|
| Water | A |
| Methanol | A |
| Isopropanol | A |
| n-Propanol | A |
| 1,2-Propanediol | A |
| Isopentanol | A |
| Acetone | A |
| Methyl isobutyl ketone | A |
| Ethyl acetate | A |
| Isopropyl acetate | A |
| Methyl tert-butyl ether | A |
| Propylene glycol methyl ether | A |
| Isopropyl ether | A |
| Tetrahydrofuran | A |
| n-Heptane | A |
| Cyclohexane | A |
| Toluene | A |
| p-Xylene | A |

**Table 3. XRPD characteristic diffraction peak data for crystal form A**

| Peak No. | 2*θ* value [° or degrees] | D[Å] | Relative intensity% |
|---|---|---|---|
| 1 | 10.459 | 8.45154 | 3.0 |
| 2 | 11.288 | 7.83224 | 100.0 |
| 3 | 13.364 | 6.62018 | 4.6 |
| 4 | 16.624 | 5.32846 | 19.5 |
| 5 | 17.312 | 5.11811 | 7.0 |
| 6 | 17.821 | 4.97311 | 3.4 |
| 7 | 18.251 | 4.85682 | 30.9 |
| 8 | 19.639 | 4.51679 | 17.0 |
| 9 | 20.086 | 4.41726 | 7.4 |
| 10 | 21.140 | 4.19929 | 7.8 |
| 11 | 22.547 | 3.94026 | 46.7 |
| 12 | 23.439 | 3.79236 | 11.5 |
| 13 | 24.234 | 3.66964 | 7.0 |
| 14 | 24.666 | 3.60644 | 3.9 |
| 15 | 26.085 | 3.41332 | 21.0 |
| 16 | 26.497 | 3.36120 | 9.0 |
| 17 | 27.050 | 3.29374 | 8.5 |
| 18 | 31.019 | 2.88074 | 3.7 |
| 19 | 33.753 | 2.65339 | 2.2 |
| 20 | 35.651 | 2.51632 | 4.1 |

### Example 3. Preparation of crystal form A of compound of formula 1

10 mg of the compound of formula 1 was added to 1 mL of the solvent. The mixture was stirred at 60 °C for dissolution, cooled, stirred for crystallization, centrifuged, and dried in vacuum to give a solid. The product was identified as the crystal form A by X-ray powder diffraction.

**Table 4. Solvents selected for preparing crystal form A by cooling crystallization and XRPD results**

| Solvent | Crystal form |
|---|---|
| Acetonitrile | A |
| Acetonitrile/methanol (1:1) | A |
| Benzyl alcohol | A |
| Nitromethane | A |
| Dimethylacetamide | A |

### Example 4. Preparation of crystal form A of compound of formula 1

10 mg of the compound of formula 1 was added to 0.2 mL of dimethyl sulfoxide for dissolution. After 1 mL of a solvent was added, a precipitate was observed. The mixture was centrifuged and dried in vacuum to give a solid. The crystal form of the resulting solid was determined by X-ray powder diffraction, as shown in Table 5 below.

**Table 5. Solvents selected for preparing crystal form A by antisolvent crystallization and XRPD results**

| Solvent | Crystal form |
|---|---|
| Water | A |
| Methanol | A |
| Isopropanol | A |
| Acetonitrile | A |
| Isopropyl ether | A |
| Ethyl acetate | A |

### Example 5. Preparation of crystal form A of compound of formula 1

10 mg of the compound of formula 1 was added to 0.2 mL of dimethyl sulfoxide for dissolution. After 1 mL of acetone was added, no precipitate was observed. The mixture was cooled to 5 °C, stirred for crystallization, centrifuged, and dried in vacuum to give a solid. The product was identified as the crystal form A by X-ray powder diffraction.

### Example 6. Preparation of crystal form A of compound of formula 1

10 mg of the compound of formula 1 was added to 0.3 mL of dichloromethane for dissolution. After 1 mL of a solvent was added, a precipitate was observed. The mixture was centrifuged and dried in vacuum to give a solid. The crystal form of the resulting solid was determined by X-ray powder diffraction, as shown in Table 6 below.

**Table 6. Solvents selected for preparing crystal form A by antisolvent crystallization and XRPD results**

| Solvent | Crystal form |
|---|---|
| Methanol | A |
| Ethanol | A |
| Acetone | A |
| Methyl tert-butyl ether | A |
| Isopropanol | A |
| Methyl isobutyl ketone | A |
| Toluene | A |
| Isopropyl ether | A |
| Ethyl acetate | A |

### Example 7. Preparation of crystal form B of compound of formula 1

10 mg of the compound of formula 1 was added to 0.2 mL of dimethyl sulfoxide for dissolution. 1 mL of methyl tert-butyl ether, and the mixture was stirred and triturated at room temperature for crystallization, centrifuged, and dried in vacuum to give a solid. The product was identified by X-ray powder diffraction as crystal form B. The XRPD pattern is shown in FIG. 2, with its characteristic peak positions listed in Table 7. The DSC profile shows endothermic peaks at 79.87 °C and 240.13 °C. The TGA profile shows a weight loss of 2.43% from 30 °C to 130 °C.

**Table 7. XRPD characteristic diffraction peak data for crystal form B**

| Peak No. | 2*θ* value [° or degrees] | D[Å] | Relative intensity% |
|---|---|---|---|
| 1 | 6.454 | 13.68343 | 14.6 |
| 2 | 7.100 | 12.44109 | 60.3 |
| 3 | 7.891 | 11.19464 | 3.6 |
| 4 | 10.448 | 8.46011 | 8.2 |
| 5 | 11.249 | 7.85922 | 100.0 |
| 6 | 13.346 | 6.62911 | 6.1 |
| 7 | 15.965 | 5.54676 | 7.7 |
| 8 | 16.595 | 5.33767 | 9.8 |
| 9 | 17.349 | 5.10747 | 4.7 |
| 10 | 18.241 | 4.85967 | 20.0 |
| 11 | 19.637 | 4.51714 | 13.3 |
| 12 | 20.092 | 4.41590 | 18.0 |
| 13 | 20.407 | 4.34837 | 17.5 |
| 14 | 21.280 | 4.17194 | 8.3 |
| 15 | 22.580 | 3.93465 | 30.4 |
| 16 | 23.470 | 3.78736 | 12.6 |
| 17 | 24.197 | 3.67517 | 7.0 |
| 18 | 24.641 | 3.60997 | 7.2 |
| 19 | 26.107 | 3.41044 | 20.2 |
| 20 | 26.550 | 3.35457 | 10.1 |
| 21 | 27.069 | 3.29143 | 4.8 |
| 22 | 30.940 | 2.88790 | 3.7 |

### Example 8. Preparation of crystal form C of compound of formula 1

10 mg of the compound of formula 1 was added to 0.3 mL of dichloromethane for dissolution. 1 mL of tetrahydrofuran was added. The mixture was cooled to 5 °C, stirred for crystallization, centrifuged, and dried in vacuum to give a solid. The product was identified by X-ray powder diffraction as crystal form C. The XRPD pattern is shown in FIG. 3, with its characteristic peak positions listed in Table 8. The DSC profile shows endothermic peaks at 99.14 °C and 240.84 °C. The TGA profile shows a weight loss of 10.28% from 30 °C to 130 °C.

**Table 8. XRPD characteristic diffraction peak data for crystal form C**

| Peak No. | 2*θ* value [° or degrees] | D[Å] | Relative intensity% |
|---|---|---|---|
| 1 | 6.484 | 13.61987 | 19.3 |
| 2 | 7.056 | 12.51794 | 100.0 |
| 3 | 7.907 | 11.17242 | 6.1 |
| 4 | 11.060 | 7.99328 | 49.9 |
| 5 | 12.702 | 6.96346 | 1.3 |
| 6 | 14.223 | 6.22214 | 6.3 |
| 7 | 15.973 | 5.54405 | 3.8 |
| 8 | 17.526 | 5.05610 | 1.8 |
| 9 | 20.342 | 4.36207 | 14.8 |
| 10 | 21.460 | 4.13733 | 2.1 |
| 11 | 23.090 | 3.84887 | 3.9 |
| 12 | 25.722 | 3.46073 | 0.9 |
| 13 | 26.398 | 3.37355 | 2.1 |
| 14 | 27.500 | 3.24082 | 1.2 |
| 15 | 31.646 | 2.82507 | 0.8 |

### Example 9. Preparation of crystal form C of compound of formula 1

10 mg of the compound of formula 1 was added to 0.3 mL of trichloromethane. The mixture was stirred at 60 °C for dissolution, cooled, stirred for crystallization, centrifuged, and dried in vacuum to give a solid. The product was identified as the crystal form C by X-ray powder diffraction.

### Example 10. Preparation of crystal form D of compound of formula 1

10 mg of the compound of formula 1 was added to 0.25 mL of N,N-dimethylformamide. The mixture was stirred at 60 °C for dissolution, cooled, stirred for crystallization, centrifuged, and dried in vacuum to give a solid. The product was identified by X-ray powder diffraction as crystal form D. The XRPD pattern is shown in FIG. 4, with its characteristic peak positions listed in Table 9. The DSC profile shows endothermic peaks at 104.54 °C and 240.90 °C. A TGA profile shows a weight loss of 11.10% from 30 °C to 200 °C.

**Table 9. XRPD characteristic diffraction peak data for crystal form D**

| Peak No. | 2*θ* value [° or degrees] | D[Å] | Relative intensity% |
|---|---|---|---|
| 1 | 6.504 | 13.57901 | 26.3 |
| 2 | 7.051 | 12.52584 | 100.0 |
| 3 | 7.896 | 11.18801 | 12.0 |
| 4 | 11.086 | 7.97447 | 86.7 |
| 5 | 13.560 | 6.52470 | 6.8 |
| 6 | 14.194 | 6.23466 | 7.1 |
| 7 | 15.876 | 5.57793 | 11.8 |
| 8 | 16.844 | 5.25946 | 1.1 |
| 9 | 17.504 | 5.06256 | 6.6 |
| 10 | 18.599 | 4.76672 | 10.4 |
| 11 | 20.238 | 4.38426 | 25.8 |
| 12 | 20.633 | 4.30125 | 8.8 |
| 13 | 21.428 | 4.14347 | 2.7 |
| 14 | 22.244 | 3.99319 | 7.1 |
| 15 | 23.175 | 3.83490 | 5.7 |
| 16 | 23.590 | 3.76843 | 4.4 |
| 17 | 23.963 | 3.71063 | 9.3 |
| 18 | 24.890 | 3.57446 | 3.0 |
| 19 | 25.628 | 3.47320 | 1.9 |
| 20 | 26.487 | 3.36238 | 6.1 |
| 21 | 27.228 | 3.27264 | 3.8 |
| 22 | 31.460 | 2.84132 | 2.9 |

### Example 11. Preparation of crystal form E of compound of formula 1

10 mg of the compound of formula 1 was added to 0.6 mL of 1,2-dichloroethane. The mixture was stirred at 60 °C for dissolution, cooled, stirred for crystallization, centrifuged, and dried in vacuum to give a solid. The product was identified by X-ray powder diffraction as crystal form E. The XRPD pattern is shown in FIG. 5, with its characteristic peak positions listed in Table 10. The DSC profile shows endothermic peaks at 113.17 °C and 241.21 °C. The TGA profile shows a weight loss of 11.13% from 30 °C to 130 °C.

**Table 10. XRPD characteristic diffraction peak data for crystal form E**

| Peak No. | 2*θ* value [° or degrees] | D[Å] | Relative intensity% |
|---|---|---|---|
| 1 | 6.490 | 13.60840 | 15.1 |
| 2 | 7.069 | 12.49555 | 100.0 |
| 3 | 8.034 | 10.99585 | 7.1 |
| 4 | 10.968 | 8.05992 | 58.1 |
| 5 | 12.949 | 6.83139 | 0.7 |
| 6 | 13.596 | 6.50778 | 1.9 |
| 7 | 14.194 | 6.23462 | 8.1 |
| 8 | 16.199 | 5.46718 | 7.4 |
| 9 | 17.328 | 5.11364 | 1.3 |
| 10 | 18.656 | 4.75232 | 4.8 |
| 11 | 20.486 | 4.33178 | 19.8 |
| 12 | 21.539 | 4.12240 | 3.3 |
| 13 | 22.360 | 3.97275 | 2.6 |
| 14 | 22.949 | 3.87215 | 5.6 |
| 15 | 23.979 | 3.70813 | 3.5 |
| 16 | 24.676 | 3.60495 | 3.0 |
| 17 | 26.411 | 3.37195 | 3.9 |
| 18 | 27.792 | 3.20747 | 1.6 |

### Example 12. Preparation of crystal form F of compound of formula 1

The compound of formula **1** (11 g, 23.4 mmol) was dispersed in 55 mL of ethyl acetate. The mixture was stirred at room temperature for 1 h and filtered. The solid was collected and dried in vacuum at 40 °C for 6 h to give a solid. The product was identified by X-ray powder diffraction as crystal form F. The XRPD pattern is shown in FIG. 6, with its characteristic peak positions listed in Table 11. The DSC profile shows endothermic peaks at 111.60 °C, 115.04 °C, and 241.06 °C. A TGA profile shows a weight loss of 9.12% from 40 °C to 180 °C.

**Table 11. XRPD characteristic diffraction peak data for crystal form F**

| Peak No. | 2*θ* value [° or degrees] | D[Å] | Relative intensity% |
|---|---|---|---|
| 1 | 7.327 | 12.0561 | 100.0 |
| 2 | 7.976 | 11.0765 | 6.1 |
| 3 | 8.690 | 10.1672 | 14.3 |
| 4 | 8.970 | 9.8505 | 11.6 |
| 5 | 9.387 | 9.4137 | 48.9 |
| 6 | 9.653 | 9.1554 | 40.7 |
| 7 | 11.843 | 7.4664 | 2.0 |
| 8 | 12.583 | 7.0289 | 20.4 |
| 9 | 13.968 | 6.3350 | 47.8 |
| 10 | 14.533 | 6.0900 | 7.7 |
| 11 | 15.793 | 5.6070 | 40.3 |
| 12 | 16.146 | 5.4852 | 9.0 |
| 13 | 16.429 | 5.3913 | 10.5 |
| 14 | 16.807 | 5.2709 | 9.3 |
| 15 | 17.270 | 5.1305 | 14.3 |
| 16 | 17.975 | 4.9309 | 9.7 |
| 17 | 20.198 | 4.3928 | 32.6 |
| 18 | 20.810 | 4.2651 | 26.1 |
| 19 | 21.385 | 4.1518 | 3.3 |
| 20 | 21.813 | 4.0711 | 41.1 |
| 21 | 22.186 | 4.0035 | 22.0 |
| 22 | 23.144 | 3.8400 | 4.7 |
| 23 | 23.410 | 3.7969 | 10.6 |
| 24 | 23.901 | 3.7200 | 6.7 |
| 25 | 24.438 | 3.6395 | 10.7 |
| 26 | 25.261 | 3.5227 | 8.4 |
| 27 | 25.444 | 3.4979 | 19.2 |
| 28 | 25.676 | 3.4668 | 9.0 |
| 29 | 27.356 | 3.2575 | 11.5 |
| 30 | 27.583 | 3.2313 | 6.5 |
| 31 | 28.071 | 3.1762 | 3.6 |
| 32 | 28.556 | 3.1234 | 4.2 |
| 33 | 29.412 | 3.0343 | 3.7 |
| 34 | 30.110 | 2.9656 | 1.6 |
| 35 | 30.577 | 2.9213 | 1.9 |
| 36 | 31.291 | 2.8563 | 1.7 |
| 37 | 31.896 | 2.8035 | 1.4 |
| 38 | 32.478 | 2.7546 | 1.4 |
| 39 | 32.892 | 2.7209 | 2.3 |
| 40 | 34.681 | 2.5845 | 1.3 |
| 41 | 36.065 | 2.4884 | 1.9 |

### Example 13. Preparation of crystal form G of compound of formula 1

The compound of formula **1** (216 mg, 0.46 mmol) was dispersed in 10 mL of dichloromethane and completely dissolved by stirring at room temperature. The solution was concentrated at reduced pressure, and the solid was retained and dried in vacuum at room temperature for 2 h to give a solid. The product was identified by X-ray powder diffraction as crystal form G. The XRPD pattern is shown in FIG. 7, with its characteristic peak positions listed in Table 12. The DSC profile shows endothermic peaks at 87.44 °C and 239.07 °C and exothermic peaks at 113.64 °C and 294.50 °C. The TGA profile shows a weight loss of 2.51% from 40 °C to 150 °C and a weight loss of 6.48% from 150 °C to 280 °C.

**Table 12. XRPD characteristic diffraction peak data for crystal form G**

| Peak No. | 2*θ* value [° or degrees] | D[Å] | Relative intensity% |
|---|---|---|---|
| 1 | 6.614 | 13.3540 | 50.60 |
| 2 | 7.192 | 12.2811 | 100.00 |
| 3 | 8.164 | 10.8219 | 41.40 |
| 4 | 9.417 | 9.3838 | 9.40 |
| 5 | 10.700 | 8.2619 | 6.00 |
| 6 | 11.113 | 7.9551 | 93.30 |
| 7 | 11.377 | 7.7715 | 15.60 |
| 8 | 12.266 | 7.2103 | 7.70 |
| 9 | 12.978 | 6.8159 | 14.60 |
| 10 | 13.738 | 6.4408 | 8.40 |
| 11 | 14.360 | 6.1629 | 14.30 |
| 12 | 16.302 | 5.4331 | 26.90 |
| 13 | 18.317 | 4.8397 | 6.30 |
| 14 | 18.807 | 4.7145 | 8.30 |
| 15 | 19.726 | 4.4969 | 3.00 |
| 16 | 20.560 | 4.3163 | 37.50 |
| 17 | 21.639 | 4.1036 | 10.70 |
| 18 | 22.588 | 3.9333 | 16.80 |
| 19 | 23.042 | 3.8567 | 16.70 |
| 20 | 24.186 | 3.6768 | 16.90 |
| 21 | 24.981 | 3.5617 | 8.30 |
| 22 | 26.080 | 3.4140 | 9.60 |
| 23 | 26.521 | 3.3583 | 12.10 |
| 24 | 28.740 | 3.1037 | 3.90 |
| 25 | 31.986 | 2.7958 | 2.80 |
| 26 | 32.900 | 2.7202 | 2.30 |
| 27 | 33.818 | 2.6484 | 2.80 |
| 28 | 40.294 | 2.2364 | 1.80 |

### Example 14. Preparation of crystal form H of compound of formula 1

100 mg of the compound of formula 1 was dissolved in 2 mL of a 2 M aqueous sodium hydroxide solution and 3 mL of purified water. The mixture was stirred at 37 °C for 1 h to precipitate a solid and centrifuged, and the supernatant was discarded. Then 5 mL of a 0.1 M aqueous hydrochloric acid solution was added, and the mixture was suspended and triturated at 37 °C, centrifuged, and dried in vacuum to give a solid. The product was identified by X-ray powder diffraction as crystal form H. The XRPD pattern is shown in FIG. 8, with its characteristic peak positions listed in Table 13. The DSC profile shows an endothermic peak at 240.45 °C. The TGA profile shows a weight loss of 0.20% from 30 °C to 233 °C.

**Table 13. XRPD characteristic diffraction peak data for crystal form H**

| Peak No. | 2*θ* value [° or degrees] | D[Å] | Relative intensity% |
|---|---|---|---|
| 1 | 5.271 | 16.75065 | 100.0 |
| 2 | 10.587 | 8.34929 | 88.2 |
| 3 | 11.892 | 7.43624 | 24.5 |
| 4 | 13.230 | 6.68686 | 34.2 |
| 5 | 15.393 | 5.75164 | 6.3 |
| 6 | 16.017 | 5.52903 | 80.9 |
| 7 | 16.625 | 5.32828 | 19.7 |
| 8 | 17.268 | 5.13123 | 7.1 |
| 9 | 19.110 | 4.64055 | 25.1 |
| 10 | 20.878 | 4.25135 | 13.1 |
| 11 | 21.498 | 4.13010 | 76.1 |
| 12 | 22.753 | 3.90507 | 61.4 |
| 13 | 24.080 | 3.69285 | 36.9 |
| 14 | 25.459 | 3.49578 | 16.9 |
| 15 | 26.175 | 3.40185 | 21.9 |
| 16 | 26.938 | 3.30710 | 59.9 |
| 17 | 27.536 | 3.23663 | 28.3 |
| 18 | 30.182 | 2.95871 | 5.8 |
| 19 | 31.015 | 2.88110 | 15.3 |
| 20 | 31.694 | 2.82092 | 20.0 |
| 21 | 32.264 | 2.77230 | 10.6 |
| 22 | 38.116 | 2.35909 | 17.0 |

### Example 15. Preparation of crystal form I of compound of formula 1

The compound of formula **1** (14 mg, 0.03 mmol) was dissolved in 0.5 mL of dichloromethane. The solution was filtered, and the filtrate was collected and left to stand at room temperature for 120 h to precipitate a solid. The product was identified by X-ray powder diffraction as crystal form I. The XRPD pattern is shown in FIG. 9, with its characteristic peak positions listed in Table 14. The DSC profile shows endothermic peaks at 77.76 °C and 241.38 °C and exothermic peaks at 113.29 °C. The TGA profile shows a weight loss of 7.58% from 40 °C to 200 °C.

**Table 14. XRPD characteristic diffraction peak data for crystal form I**

| Peak No. | 2*θ* value [° or degrees] | D[Å] | Relative intensity% |
|---|---|---|---|
| 1 | 5.963 | 14.8104 | 45.70 |
| 2 | 9.438 | 9.3633 | 21.10 |
| 3 | 10.303 | 8.5788 | 22.10 |
| 4 | 11.156 | 7.9247 | 12.40 |
| 5 | 11.883 | 7.4414 | 100.00 |
| 6 | 13.446 | 6.5798 | 19.40 |
| 7 | 13.953 | 6.3417 | 62.00 |
| 8 | 14.649 | 6.0420 | 2.40 |
| 9 | 15.718 | 5.6333 | 14.00 |
| 10 | 17.945 | 4.9392 | 35.10 |
| 11 | 18.356 | 4.8295 | 37.70 |
| 12 | 19.518 | 4.5444 | 94.20 |
| 13 | 19.807 | 4.4788 | 31.10 |
| 14 | 20.305 | 4.3700 | 44.90 |
| 15 | 21.559 | 4.1185 | 15.20 |
| 16 | 22.086 | 4.0215 | 96.00 |
| 17 | 23.016 | 3.8611 | 7.50 |
| 18 | 25.009 | 3.5578 | 38.10 |
| 19 | 26.043 | 3.4188 | 14.00 |
| 20 | 26.521 | 3.3582 | 13.00 |
| 21 | 27.570 | 3.2328 | 14.50 |
| 22 | 28.478 | 3.1318 | 2.10 |
| 23 | 28.967 | 3.0799 | 13.20 |
| 24 | 29.576 | 3.0179 | 19.30 |
| 25 | 30.192 | 2.9577 | 17.70 |
| 26 | 30.955 | 2.8865 | 5.00 |
| 27 | 31.936 | 2.8001 | 5.60 |
| 28 | 33.498 | 2.6730 | 13.80 |
| 29 | 34.891 | 2.5694 | 3.30 |
| 30 | 35.037 | 2.5590 | 2.90 |
| 31 | 35.787 | 2.5071 | 4.50 |
| 32 | 36.276 | 2.4744 | 6.40 |
| 33 | 37.592 | 2.3908 | 3.40 |
| 34 | 39.562 | 2.2762 | 9.10 |
| 35 | 40.042 | 2.2500 | 4.20 |
| 36 | 40.966 | 2.2013 | 2.30 |
| 37 | 41.490 | 2.1747 | 1.60 |
| 38 | 42.429 | 2.1287 | 2.40 |
| 39 | 43.344 | 2.0859 | 1.90 |
| 40 | 45.068 | 2.0100 | 2.70 |

### Example 16. Hygroscopicity study of crystal forms

Surface Measurement Systems intrinsic DVS was adopted. At 25 °C, the crystal forms were tested in a humidity range of 0%-95%, with increments of 10%. The criterion was that each gradient mass change dM/dT is less than 0.002%, TMAX 360 min, two cycles.

**Table 15**

| Crystal form | 0.0%RH-80.0%RH | Crystal form |
|---|---|---|
| Crystal form A | 0.10% | No change |
| Crystal form H | 19.91% | No change |

### Example 17. Stability influencing factor study of crystal forms

Free-state samples were spread out, left to stand in open flasks, and subjected to 30-day stability tests in light exposure (4500 Lux), high temperature (40 °C and 60 °C), and high humidity (RH 75% and RH 92.5%) conditions.

**Table 16**

| Conditions | Time (days) | Purity% | Crystal form |
|---|---|---|---|
| Initial | 0 | 99.1 | A |
| Light exposure (4500 Lux) | 7 | 99.0 | A |
| | 14 | 98.9 | A |
| | 30 | 98.9 | A |
| 40 °C | 5 | 99.1 | A |
| | 10 | 99.1 | A |
| | 30 | 99.1 | A |
| 60 °C | 7 | 99.1 | A |
| | 14 | 99.1 | A |
| | 30 | 99.1 | A |
| 75% RH | 7 | 99.1 | A |
| | 14 | 99.1 | A |
| | 30 | 99.1 | A |
| 92.5% RH | 7 | 99.1 | A |
| | 14 | 99.1 | A |
| | 30 | 99.1 | A |

The results of the influencing factor study show that the crystal form A has good physical and chemical stability at high temperature, high humidity, and light exposure.

### Example 18: Long-term/accelerated stability of crystal forms

Free-state samples were sealed in aluminum foil bags and left to stand in 25 °C/60% RH and 40 °C/75% RH conditions to test their stability. The results are shown below.

**Table 17**

| | Standing conditions | Purity% | | | | | | Crystal form |
|---|---|---|---|---|---|---|---|---|
| Crystal form A | | Initial | 14 days | 1 month | 2 months | 3 months | 9 months | |
| | 25 °C/60%RH | 99.1 | 99.1 | 99.1 | 99.1 | 99.1 | 99.1 | No change |
| | 40 °C/75%RH | | 99.1 | 99.1 | 99.1 | 99.1 | 99.1 | No change |

The experimental results show that: standing in long-term accelerated conditions for 9 months, crystal form A exhibited good physical stability and chemical stability.

## Claims

1. A crystal form A of the compound of formula 1, comprising an X-ray powder diffraction pattern, represented by diffraction angles 2*θ*, having characteristic peaks at 11.288, 16.624, 18.251, 19.639, 22.547, and 26.085, preferably characteristic peaks at 11.288, 16.624, 18.251, 19.639, 21.140, 22.547, 23.439, 26.085, 26.497, and 27.050, and more preferably characteristic peaks at 11.288, 16.624, 17.312, 18.251, 19.639, 20.086, 21.140, 22.547, 23.439, 24.234, 26.085, 26.497, and 27.050,

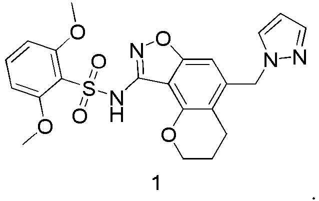

2. The crystal form A according to claim 1, wherein the X-ray powder diffraction pattern represented by diffraction angles 2*θ* is shown in FIG. 1.

3. A method for preparing the crystal form A according to claim 1 or 2, wherein the method is selected from the group consisting of:
method I, comprising: adding the compound of formula 1 to a solvent (I), and triturating for crystallization, wherein the solvent (I) is one or more solvents selected from the group consisting of an alcohol solvent, a ketone solvent, an ester solvent, an ether solvent, a hydrocarbon solvent, a nitrile solvent, water, and dimethyl sulfoxide;
the alcohol solvent is selected from the group consisting of methanol, ethanol, isopropanol, n-propanol, benzyl alcohol, 1,2-propanediol, and isopentanol;
the ketone solvent is selected from the group consisting of acetone and methyl isobutyl ketone;
the ester solvent is selected from the group consisting of ethyl acetate and isopropyl acetate;
the ether solvent is selected from the group consisting of methyl tert-butyl ether, propylene glycol methyl ether, isopropyl ether, and tetrahydrofuran;
the hydrocarbon solvent is selected from the group consisting of nitromethane, n-heptane, cyclohexane, toluene, and p-xylene;
the nitrile solvent is selected from the group consisting of acetonitrile;
method II, comprising: dissolving the compound of formula 1 in a solvent (II) by heating, and stirring at room temperature or at a reduced temperature for crystallization, wherein the solvent (II) is one or more solvents selected from the group consisting of acetonitrile, acetone, nitromethane, benzyl alcohol, N,N-dimethylacetamide, dimethyl sulfoxide, N-methylpyrrolidone, acetonitrile/methanol, and 1,2-dichloroethane; and
method III, comprising: dissolving the compound of formula 1 in a solvent (III), adding a solvent (IV), and stirring for crystallization, wherein the solvent (III) is selected from the group consisting of dimethyl sulfoxide and dichloromethane; the solvent (IV) is one or more solvents selected from the group consisting of water, methanol, ethanol, isopropanol, isopropyl ether, methyl tert-butyl ether, acetone, methyl isobutyl ketone, acetonitrile, ethyl acetate, and toluene.

4. A crystal form B of the compound of formula 1, comprising an X-ray powder diffraction pattern, represented by diffraction angles 2*θ*, having characteristic peaks at 7.100, 11.249, 18.241, 22.580, and 26.107, preferably characteristic peaks at 6.454, 7.100, 11.249, 18.241, 19.637, 20.092, 20.407, 22.580, 26.107, and 26.550, and more preferably characteristic peaks at 6.454, 7.100, 10.448, 11.249, 16.595, 18.241, 19.637, 20.092, 20.407, 22.580, 23.470, 26.107, and 26.550.

5. The crystal form B according to claim 4, wherein the X-ray powder diffraction pattern represented by diffraction angles 2*θ* is shown in FIG. 2.

6. A method for preparing the crystal form B according to claim 4 or 5, comprising:
dissolving the compound of formula 1 in a solvent (V), adding a solvent (VI), and stirring for crystallization, wherein the solvent (V) is selected from the group consisting of dimethyl sulfoxide; the solvent (VI) is selected from the group consisting of methyl tert-butyl ether.

7. A crystal form C of the compound of formula 1, comprising an X-ray powder diffraction pattern, represented by diffraction angles 2*θ*, having characteristic peaks at 6.484, 7.056, 7.907, 11.060, 14.223, and 20.342, preferably characteristic peaks at 6.484, 7.056, 7.907, 11.060, 14.223, 15.973, 20.342, 21.460, 23.090, and 26.398, and more preferably characteristic peaks at 6.484, 7.056, 7.907, 11.060, 12.702, 14.223, 15.973, 17.526, 20.342, 21.460, 23.090, 26.398, and 27.500.

8. The crystal form C according to claim 7, wherein the X-ray powder diffraction pattern represented by diffraction angles 2*θ* is shown in FIG. 3.

9. A method for preparing the crystal form C according to claim 7 or 8, wherein the method is selected from the group consisting of:
method I, comprising: dissolving the compound of formula 1 in a solvent (VII), and adding a solvent (VIII) for antisolvent crystallization, wherein the solvent (VII) is selected from the group consisting of dichloromethane and tetrahydrofuran, and the solvent (VIII) is one or more solvents selected from the group consisting of tetrahydrofuran and n-hexane; and
method II, comprising: dissolving the compound of formula 1 in a solvent (IX), and stirring for crystallization, wherein the solvent (IX) is selected from the group consisting of trichloromethane.

10. A crystal form D of the compound of formula 1, comprising an X-ray powder diffraction pattern, represented by diffraction angles 2*θ*, having characteristic peaks at 6.504, 7.051, 7.896, 11.086, 15.876, and 20.238, preferably characteristic peaks at 6.504, 7.051, 7.896, 11.086, 13.560, 14.194, 15.876, 17.504, 18.599, 20.238, 22.244, and 23.963, and more preferably characteristic peaks at 6.504, 7.051, 7.896, 11.086, 13.560, 14.194, 15.876, 17.504, 18.599, 20.238, 20.633, 22.244, 23.963, and 26.487.

11. The crystal form D according to claim 10, wherein the X-ray powder diffraction pattern represented by diffraction angles 2*θ* is shown in FIG. 4.

12. A method for preparing the crystal form D according to claim 10 or 11, comprising:
dissolving the compound of formula 1 in a solvent (X), and stirring for crystallization, wherein the solvent (X) is selected from the group consisting of N,N-dimethylformamide.

13. A crystal form E of the compound of formula 1, comprising an X-ray powder diffraction pattern, represented by diffraction angles 2*θ*, having characteristic peaks at 6.490, 7.069, 10.968, 14.194, 16.199, and 20.486, preferably characteristic peaks at 6.490, 7.069, 8.034, 10.968, 14.194, 16.199, 18.656, 20.486, 21.539, and 22.949, and more preferably characteristic peaks at 6.490, 7.069, 8.034, 10.968, 14.194, 16.199, 18.656, 20.486, 21.539, 22.360, 22.949, 23.979, 24.676, and 26.411.

14. The crystal form E according to claim 13, wherein the X-ray powder diffraction pattern represented by diffraction angles 2*θ* is shown in FIG. 5.

15. A method for preparing the crystal form E according to claim 13 or 14, comprising:
dissolving the compound of formula 1 in a solvent (XI), and stirring for crystallization, wherein the solvent (XI) is selected from the group consisting of 1,2-dichloroethane.

16. A crystal form F of the compound of formula 1, comprising an X-ray powder diffraction pattern, represented by diffraction angles 2*θ*, having characteristic peaks at 7.327, 9.387, 13.968, 15.793, 20.198, and 21.813, preferably characteristic peaks at 7.327, 8.690, 9.387, 9.653, 12.583, 13.968, 15.793, 17.270, 20.198, 20.810, 21.813, 22.186, and 25.444, and more preferably characteristic peaks at 7.327, 8.690, 8.970, 9.387, 9.653, 12.583, 13.968, 15.793, 16.429, 17.270, 17.975, 20.198, 20.810, 21.813, 22.186, 23.410, 24.438, 25.444, and 27.356.

17. The crystal form F according to claim 16, wherein the X-ray powder diffraction pattern represented by diffraction angles 2*θ* is shown in FIG. 6.

18. A method for preparing the crystal form F according to claim 16 or 17, comprising:
dissolving the compound of formula 1 in a solvent (XII), and stirring for crystallization, wherein the solvent (XII) is selected from the group consisting of ethyl acetate.

19. A crystal form H of the compound of formula 1, comprising an X-ray powder diffraction pattern, represented by diffraction angles 2*θ*, having characteristic peaks at 5.271, 10.587, 13.230, 16.017, 21.498, 22.753, and 26.938, preferably characteristic peaks at 5.271, 10.587, 11.892, 13.230, 16.017, 16.625, 19.110, 21.498, 22.753, 24.080, 26.938, and 27.536, and more preferably characteristic peaks at 5.271, 10.587, 11.892, 13.230, 16.017, 16.625, 19.110, 21.498, 22.753, 24.080, 25.459, 26.175, 26.938, 27.536, 31.694, and 38.116.

20. The crystal form H according to claim 19, wherein the X-ray powder diffraction pattern represented by diffraction angles 2*θ* is shown in FIG. 8.

21. A method for preparing the crystal form H according to claim 19 or 20, comprising:
step a, dissolving the compound of formula 1 in an aqueous sodium hydroxide solution and stirring; and
step b, adding an aqueous hydrochloric acid solution to the solid obtained in step a and triturating for crystallization.

22. The crystal form according to any one of claims 1-2, 4-5, 7-8, 10-11, 13-14, 16-17 and 19-20, wherein the 2*θ* angles have a margin of error of ±0.20.

23. A pharmaceutical composition, comprising the crystal form according to any one of claims 1-2, 4-5, 7-8, 10-11, 13-14, 16-17, and 19-20, and optionally a pharmaceutically acceptable excipient.

24. A method for preparing a pharmaceutical composition, comprising: mixing the crystal form according to any one of claims 1-2, 4-5, 7-8, 10-11, 13-14, 16-17, and 19-20 with a pharmaceutically acceptable excipient.

25. Use of the crystal form according to any one of claims 1-2, 4-5, 7-8, 10-11, 13-14, 16-17, and 19-20 or the composition according to claim 23 in the manufacture of a medicament for preventing and/or treating cancer.
